# EUROPÄISCHE PATENTSCHRIFT

(11) **EP 2 083 911 B1**
(45) Veröffentlichungstag und Bekanntmachung des Hinweises auf die Patenterteilung: **04.01.2012**
(21) Anmeldenummer: 07818518.8
(22) Anmeldetag: 27.09.2007
(51) Int. Cl.: A61N 1/05, A61N 1/36

(54) **RETINA-IMPLANTAT MIT EINEM GRUNDKÖRPER**
RETINAL IMPLANT WITH A BASE BODY
IMPLANT RETINIEN COMPRENANT UN CORPS PRINCIPAL

(30) Priorität: 10.10.2006 DE 102006048819
(43) Veröffentlichungstag der Anmeldung: 05.08.2009
(73) Patentinhaber: Retina Implant AG, 72770 Reutlingen (DE)
(72) Erfinder: STETT, Alfred, 72770 Reutlingen (DE); NISCH, Wilfried, 72070 Tübingen (DE)
(74) Vertreter: Otten, Hajo
(86) Internationale Anmeldenummer: PCT/EP2007/008435
(87) Internationale Veröffentlichungsnummer: WO 2008/043439

(56) Entgegenhaltungen:
- EP-A- 0 388 480
- EP-A- 0 761 254
- WO-A-87/07825
- WO-A-2005/000395
- WO-A-2005/070495
- DE-A1- 10 028 522
- US-A1- 2003 100 823

## Beschreibung

Die vorliegende Erfindung betrifft ein aktives Retina-Implantat mit einer Vielzahl von Bildzellen, die einfallendes Licht in elektrische Signale umwandeln, die über Elektroden an umgebendes Gewebe abgegeben werden, wobei die Elektroden Nadelelektroden sind, die als Array auf einem Grundkörper angeordnet sind.

Ein solches Retina-Implantat ist beschrieben in D. Palanker et al.: "DESIGN OF A HIGH-RESOLUTION OPTOELECTRONIC RETINAL PROSTHESIS" in I. Neural Eng. (2005) 2 (1). Seiten 105-120.

Ein aktives Retina-Implantat ist beispielsweise auch aus der WO 2005/000395 A1 bekannt, deren Offenbarung hiermit ausdrücklich in die vorliegenden Anmeldung einbezogen wird. Dieses Retina-Implantat wird über eingestrahltes IR-Licht oder über induktiv eingekoppelte HF-Energie kabellos mit elektrischer Energie versorgt, wobei in dieser extern zugeführten Fremdenergie Informationen zur Steuerung des Implantates enthalten sein können.

Da derartige kabellose Retina-Implantate für Anwendungen am Menschen jedoch nicht mit einer zufriedenstellenden Qualität zur Verfügung stehen, werden zurzeit nicht nur epiretinale sondern auch subretinale Implantate vorgeschlagen, denen die erforderliche Fremdenergie über Kabel zugeführt wird,

So beschreiben Gekeler et al.: "COMPOUND SUBRETINAL PROSTHESES WITH EXTRA-OCULAR PARTS DESIGNED FOR HUMAN TRIALS: SUCCESSFUL LONG-TERM IMPLANTATION IN PIGS". Graefe's Arch Clin Exp Ophthalmol (28. April 2006) [Epub ahead of print], ein subretinales Retina-Implantat, bei dem die Fremdenergie und erforderliche Steuersignale kabelgebunden zu dem in das Auge implantierten Chip geleitet werden. Auch die Offenbarung dieser Veröffentlichungung wird ausdrücklich zum Gegenstand der vorliegenden Anmeldung gemacht.

Auch Sachs et al., a.a.O. beschreiben Verfahren zur subretinalen Implantation, bei denen Folienelektroden in die Netzhaut, also zwischen Pigmentepitel und neuronale Netzhaut eingeschoben werden. Hierbei muss besonders darauf geachtet werden, dass das Implantat während des Einführens nicht geschädigt wird und dass die Netzhaut durch das mechanische Einschieben zwischen die Zellschichten nicht verletzt wird.

Aus der EP 0 388 480 A1 ist eine implantierbare, poröse Reizelektrode an einem Herzschrittmacher bekannt, bei dem die Elektrodenoberfläche mit einem dünnen Überzug aus einem hydrophilen Polymer versehen ist, und in das Polymer ein entzündungshemmendes Steroid eingebettet ist. Auf diese Weise wird bei dem bekannten Herzschrittmacher verhindert, dass die Reizschwellen nach der Implantation zunächst ansteigen, was auf Entzündungsreaktionen und Vemarbungen im Bereich der Elektroden zurückzuführen ist.

Bei dem bekannten Herzschrittmacher diffundiert das Steroid aus der dünnen Schutzschicht in das angrenzende Gewebe, wodurch mögliche Entzündungsprozesse unterdrückt werden und der Einheilungsprozess der Elektrode in das Trabekelwerk des Herzmuskels unterstützt wird. Die Schutzschicht ist chemisch und thermisch stabil und hat sich als gewebe- und körperverträglich erwiesen, so dass sie bei einer langen Verweilzeit des Implantates im Körper für eine dauerhafte Abdeckung der porösen Oberfläche der Elektrode dient und diese vor Verunreinigungen schützt, wobei ein Kapazitätsverlust der porösen Elektrodenoberfläche nicht auftritt.

Retina-Implantate und Herzschrittmacher erfordern eine stabile, langzeitbeständige und funktionelle Kopplung des elektronischen mit dem biologischen System. Dies wird üblicherweise durch eine möglichst enge mechanische Kopplung der Elektroden mit dem umgebenden Gewebe erreicht, wobei die Elektrodenoberfläche häufig mit einer porösen Struktur mit möglichst großer innerer Oberfläche ausgestattet wird, so dass sie eine möglichst große Ladungsübertragungskapazität über die Helmholtz-Doppelschicht an der Grenzfläche zwischen Elektrode und im Gewebe vorhandenen Elektrolyt besitzen.

Bei Implantaten mit einer großen Elektrodendichte, wie beispielsweise bei Retina-Implantaten, ist die Fläche der einzelnen Elektroden jedoch so klein, dass auch bei einer porösen Struktur die Ladungsübertragungskapazität nicht immer ausreicht, sondern dass elektrochemische Effekte an der Elektrode auftreten, die die Stimulationsschwellen erhöhen und sogar zelltoxisch oder inflammatorisch wirken können.

Die enge mechanische Kopplung mit den zu stimulierenden Zielzellen ist bei planaren Mikroelektroden, die nicht in das angrenzende Gewebe eindringen können, darüber hinaus eingeschränkt. Insbesondere dann, wenn die Elektroden noch durch nichterregbare Zellschichten von den Zielzellen isoliert sind, kann das die Stimulationsschwellen erheblich erhöhen. Dieses Problem tritt insbesondere bei Retina-Implantaten auf, wo beispielsweise die abgestorbenen Photorezeptoren oder mögliche Glianarben den engen mechanischen Kontakt zwischen den Elektroden und den zu stimulierenden Neuronen behindern.

Um die Elektroden näher an die zu stimulierenden Zellen heranzubringen, die sich innerhalb des Gewebevolumens befinden, werden daher im Stand der Technik Nadelelektroden vorgeschlagen, die in das Gewebe eindringen sollen. Auf diese Weise werden die zum Erreichen der erforderlichen Reizschwellen benötigten Reizstärken verringert und die Ortsauflösung erhöht.

R.A. Normann: "APPLICATIONS OF PENETRATING MICROELECTRODE ARRAYS IN NERVOUS SYSTEM DISORDERS", in Review of North American Research on Brain Computer Interfaces, WTEC Workshop (2006), Seiten 66-69 beschreibt in diesem Zusammenhang die Verwendung von penetrierenden Elektroden, um besonders selektive Verbindungen zu kleinen Gruppen von Nervenzellen innerhalb von Geweben herzustellen.

Die eingangs erwähnte Veröffentlichung von D. Palanker et al. beschreibt die Verwendung von penetrierenden Elektroden bei subretinalen Implantaten. Die dabei eingesetzten Elektroden haben einen Durchmesser im Bereich von 10 µm und eine Höhe über dem Grundkörper im Bereich von 70 µm. Die Autoren konnten zeigen, dass nach einer Implantation eines Arrays von derartigen Nadelelektroden in den subretinalen Raum einer Ratte eine selbständige Migration der Zellen in die Zwischenräume zwischen den Elektroden zu beobachten war.

Bei dem Einsatz von Nadelelektroden ist es weiter sowohl bei deren Verwendung auf Implantaten als auch im Zusammenhang mit *ex vivo* zu verwendenden Vorrichtungen von besonderer Bedeutung, dass die Nadelspitzen beim Eindringen in das Gewebe keine Zellen innerhalb des Gewebeverbundes verletzen.

W. Shain et al.: "IT'S ALL IN THE SEEING: A BRIEF REVIEW OF THE STUDY OF BRAIN RESPONSES TO INSERTED NEURAL PROSTHETIC DEVICES", in Review of North American Research on Brain Computer Interfaces, WTEC Workshop (2006), Seiten 96-98 weisen in diesem Zusammenhang darauf hin, dass der wichtigste Faktor die Geschwindigkeit ist, mit der die Nadeln in das Gewebe eingeführt werden.

P. J. Rousche und R. A. Normann: "A METHOD FOR PNEUMATICALLY INSERTING AN ARRAY OF PENETRATING ELECTRODES INTO CORTICAL TISSUE" in Annals of Biomedical Engineering (1992), 20, Seiten 413-422 beschreiben ein Array von 100 Nadelelektroden, die in das Gewebe eindringen sollen. Nachdem einfaches Eindrücken des Elektrodenarrays nicht erfolgreich war, wurde das Array mittels pneumatischer Beschleunigung mit einer Geschwindigkeit zwischen 1 und 11 m/s in das Gewebe eingeschossen. Die Autoren berichten, dass eine Geschwindigkeit von mindestens 8,3 m/s erforderlich war, um alle 100 Elektroden in dem Array mit einer Tiefe von 1,5 mm in Cortexgewebe sicher einzubringen.

Andererseits berichten H. Thielecke et al.: "GENTLE CELL HANDLING WITH AN ULTRA-SLOW INSTRUMENT: CREEP-MANIPULATION OF CELLS", in Microsyst Technol (2005), 11, Seiten 1230-1241, dass es möglich ist, Mikroelektroden mit Spitzendurchmessern von wenigen µm ohne Zellschädigung in ein dreidimensionales Zellaggregat einzustechen, wenn die Bewegung der Elektroden nur entsprechend langsam erfolgt. Wenn diese Geschwindigkeit im Bereich der Migrationsgeschwindigkeit der Zellen liegt, können die Zellen den vordringenden Elektroden ausweichen, so dass sie nicht zerstört werden. Die Autoren berichten, dass sich Vorschubgeschwindigkeiten im Bereich von einigen nm/s als erfolgreich erwiesen haben.

B. Niggemann et al.: "THE MINIMAL INVASIVE RETINAL IMPLANT (miRI) PROJECT: FIRST SERIES OF IMPLANTATION WITH LONG-TERM FOLLOW-UP IN NONHUMAN PRIMATES", in Invest Ophthalmol Vis Sci (2006), 47: E-Abstract, Seite 1031 beschreiben eine Anwendung dieser langsamen Gewebepenetration, bei der Elektroden in den subretinalen Raum vorgeschoben werden, ohne dass er chirurgisch eröffnet werden musste. Dazu werden Elektroden von außen auf die Sclera aufgesetzt, wobei durch einen ständigen leichten Andruck, der durch das die Elektroden überdeckende Gewebe entsteht, die Elektroden innerhalb von Wochen oder Monaten in die Sklera eindringen und langsam in den subretinalen Raum vorwandem.

Wie bereits eingangs erwähnt, kommt es nach der Implantation von Elektroden häufig zu einer Vemarbung an der Grenzfläche zwischen Elektrode und Gewebe, wodurch sich die elektrischen Eigenschaften dieser Grenzschicht nachteilig verändern. Die Narbenschicht erhöht nämlich zum einen den elektrischen Widerstand des Kontaktes, wobei sich zum anderen dadurch der Abstand zwischen der Elektrodenoberfläche und den Zielzellen vergrößert. Beides hat zur Folge, dass sich die Stimulationseffizienz verringert, was durch erhöhten Ladungsübertrag kompensiert werden muss.

Die US 2003/0100823 beschreibt ein Implantat zur Erzeugung einer neuralen chnittstelle mit dem zentralen Nervensystem, bei dem Elektroden zwischen zwei Polyimid-Schichten angeordnet und durch Öffnungen in einer der beiden Schichten von außen kontaktiert werden.

Neben den Elektroden sind Öffnungen in den Polyimid-Schichten vorgesehen, die bioaktive Spezies aufnehmen können, die dazu dienen, die Schnittstelle auszubilden.

Die US 6.360.129 B1 beschreibt eine helixförmige Elektrode, die der elektrischen Stimulation im Rahmen eines Herzschrittmachers oder Defilibrators dient. Die Elektrode weist an ihrer Spitze eine Schutzschicht auf, die beim Einschieben der Elektrode umgebendes Gewebe schützt. Die Schutzklappe ist so beschaffen, dass sie bei Kontakt mit dem Gewebe abgebaut wird.

Vor diesem Hintergrund liegt der vorliegenden Erfindung die Aufgabe zugrunde, das eingangs erwähnte Retina-Implantat derart weiterzubilden, dass es auf schonende Weise so in Kontakt mit dem Gewebe gebracht werden kann, dass die Elektrodenspitzen dicht an die Zielzellen innerhalb des Gewebes herangeführt werden, wo sie dauerhaft stabil platziert werden können.

Erfindungsgemäß wird diese Aufgabe bei dem eingangs erwähnten Retina-Implantat dadurch gelöst, dass die Elektroden von einer Schutzschicht abegdeckt sind und die Schutzschicht derart beschaffen ist, dass sie sich nach Kontakt mit dem Gewebe auf definierte Weise zumindest so weit auflöst, dass die Elektroden in unmittelbaren Kontakt mit dem Gewebe gelangten, wobei die Schutzschicht biodegradable und/oder bioresorbierbare Materialien mit einer definierten Abbaurate umfasst.

Die der Erfindung zugrunde liegende Aufgabe wird auf diese Weise vollkommen gelöst.

Die Erfinder der vorliegenden Anmeldung haben nämlich erkannt, dass eine sich selbst auflösende Schutzschicht bei der Implantation des Retina-Implantats zunächst nicht nur die Elektroden vor einer Beschädigung im Zusammenhang mit den erforderlichen Manipulationen schützt, sondern dass auch das Gewebe, mit dem die Vorrichtung in Kontakt gebracht wird, zunächst keine Schädigungen erfährt, da die möglicherweise scharfen Spitzen der Elektroden während der Anbringung durch die Schutzschicht abgedeckt sind.

Auf diese Weise ist es also möglich, dass das neue Retina-Implantat zunächst beispielsweise in ein Gewebe implantiert wird, ohne dass die Gefahr besteht, dass die Elektroden oder das Gewebe beschädigt werden.

Damit kann das erfindungsgemäße Retina-Implantat auch in das Innere von schwer zugänglichen Gewebevoluminas eingebracht werden, ohne dass es während der in der Regel aufwändigen chirurgischen Implantationen mechanisch beschädigt werden.

Unter einer Schutzschicht, die sich "auf definierte Weise auflöst", wird eine Schutzschicht verstanden, die sich gezielt, also gewollt oder auf vorbestimmte Weise, nach dem Kontakt mit dem Gewebe selbst auflöst. Mit anderen Worten, die Schutzschicht wird innerhalb eines Zeitraumes kontinuierlich abgebaut, der zwischen der Herstellung des Kontaktes zwischen Gewebe und Schutzschicht und der Inbetriebnahme der Vorrichtung liegt. Der Abbau der Schutzschicht kann dabei durch chemische, biologische oder physikalische Prozesse ausgelöst werden. Erst nachdem die Vorrichtung also an Ort und Stelle gebracht wurde, beginnt die Schutzschicht sich aufzulösen und die Elektroden werden freigegeben und gelangen allmählich in Kontakt mit dem Gewebe.

Mit der erfindungsgemäßen Ausgestaltung des Retina-Implantates ist es jetzt möglich, solche Implantate mit einem Array von hervorstehenden Nadelelektroden zu versehen, ohne dass die genannten Probleme bei der Implantation auftauchen, Nach der Implantation löst sich die Schutzschicht auf, so dass die Schichten der Retina sich langsam an die Nadelspitzen anlagern können, wobei die Nadeln mit zunehmender Auflösung des resorbierbaren Materials weiter in das Gewebe eindringen, ohne dass dadurch Zellen verletzt werden.

Die Erfinder der vorliegenden Anmeldung haben erkannt, dass viele Probleme mit der Implantation derartiger Retina-Implantate vermieden werden können, wenn die in einem Array angeordneten Nadelelektroden in eine sich nach Implantation selbst auflösende Schutzschicht eingebettet werden. Nach Implantation wird die Schicht erfindungsgemäß abgebaut und/oder resorbiert, so dass die Nadelspitzen in Kontakt mit dem Gewebe kommen. Das Material, aus dem die Schutzschicht besteht, ist dabei so ausgewählt bzw. modifiziert, dass die Abbaurate gut definiert ist. Bei dem langsamen Abbau der Schutzschicht werden die Spitzen der Nadelelektroden allmählich immer weiter von der Schutzschicht freigegeben und gelangen in Kontakt mit den Zellen, die entsprechend ihrer Migrationsgeschwindigkeit den Spitzen ausweichen. Die Nadelelektroden dringen also langsam in das Gewebe ein, ohne dieses zu verletzen. Die Penetration wird dabei dadurch unterstützt, dass das Gewebe, in das das Retina-Implantat implantiert wurde, einen Druck auf das Array ausübt, so dass eine Kraft in Richtung der Nadelachsen entsteht.

Auf diese Weise ist es jetzt auch möglich, Nadelelektroden in tiefer liegende oder schwer zugängliche Gewebeschichten vorzuschieben, ohne dass die Gefahr einer mechanischen Beschädigung der Elektroden bzw. des Gewebes besteht.

Grundsätzlich können Implantate nur von einer frei zugänglichen Oberfläche aus in die gewünschten Gewebeschichten vorgeschoben werden, wobei Elektroden, die senkrecht zur Vorschubrichtung von dem Grundkörper abstehen, durch den mechanischen Vorschub geschädigt werden können, wobei aber andererseits auch die Gefahr besteht, dass diese Elektroden das Gewebe entlang des im Gewebe liegenden Vorschubkanals verletzen.

Ferner sind für die Penetration der Nadelelektroden in das Gewebe mechanische Drücke erforderlich, die in Vorschubrichtung auf die Elektroden wirken müssen. Dies bedeutet jedoch, dass im Stand der Technik Nadelelektroden nur in Zellschichten vorgeschoben werden konnten, die von außen mit Hilfe mechanischer Hilfsmittel zugänglich waren; siehe H. G. Sachs et al.: "TRANSSCLERAL IMPLANTATION AND NEUROPHYSIOLOGICAL TESTING OF SUBRETINAL POLYIMIDE FILM ELECTRODES IN THE DOMESTIC PIG IN VISUAL PROSTHESIS DEVELOPMENT", in J. Neural Eng. (2005), 2 (1), Seiten 57-64 zeigen gängige Operationsverfahren für Retina Implantate.

Zudem sind kontrollierte Eindringgeschwindigkeiten nur schwer herzustellen, wenn die Elektroden nach intrakortikaler Lokalisation langsam in das gewünschte Zellvolumen eindringen sollen, wobei dazu unter Umständen auch tagelange chirurgische Eingriffe erforderlich wären; siehe W. Shain et al., a.a.O.

Unter einer Nadelelektrode wird im Rahmen der vorliegenden Anmeldung neben langen zylindrischen, sich zu ihrer Spitze ggf. verjüngenden Elektroden auch jede andere Art von Elektroden verstanden, die über den Grundkörper hervorstehen. Die Elektroden können dabei vollständig oder teilweise aus leitfähigen Materialien gefertigt sein, wobei beispielsweise auch nur die Spitze der Elektrode selbst leitfähig ausgebildet sein kann.

Unter einem Grundkörper wird im Rahmen der vorliegenden Erfindung beispielsweise eine flexible Folie verstanden, auf der neben den Elektroden auch noch verschiedene elektronische Komponenten aufgebracht sind. Der Grundkörper kann aber auch aus jedem anderen, auch steifen Material gefertigt sein. Schließlich ist es auch nicht erforderlich, dass auf dem Grundkörper zusätzlich zu den Elektroden noch weitere elektronische Komponenten angeordnet sind, der Grundkörper kann also lediglich das Elektrodenarray enthalten, wobei das Elektrodenarray über mehradrige Zuleitungen, beispielsweise ein Flachbandkabel, mit einer Stimulations- und/oder Messelektronik verbunden ist.

Die Elektroden haben an ihrer Spitze dabei vorzugsweise Abmessungen in der Größenordnung von zellulären Strukturen wie Axonen, Dendriten oder Zellkörpem, sie sind im Durchmesser vorzugsweise im Bereich von 1 bis 10 µm angesiedelt.

Um die Nadelelektroden genügend weit in das Gewebe vortreiben zu können, sind die Elektroden vorzugsweise nadelförmig ausgebildet und weisen Längen im Bereich von bis zu mehreren 10 µm auf.

Weil die Schutzschicht biodegradable und/oder bioresorbierbare Materialien mit einer definierten Abbaurate umfasst, ist von Vorteil, dass derartige Materialien eine geringe Abbaurate haben, so dass der langsame Abbau der Schutzschicht die kontrollierte langsame Penetration der Elektroden in das Gewebe ermöglicht, wobei der Anpressdruck durch das sozusagen auf der anderen Seite der Vorrichtung liegende Gewebe ausgeübt wird. Mit anderen Worten, die Geschwindigkeit, mit der die Elektroden, vorzugsweise die Nadeln, in das Gewebe eindringen, wird durch die Rate bestimmt, mit der die Schutzschicht abgebaut wird.

Biodegradable und bioresorbierbare Materialien sind an sich bekannt, sie werden bei der Herstellung und Verwendung von biomedizinischen Implantaten vielfach eingesetzt. Sie zeichnen sich durch ihre Biokompatibilität und ihre natürliche Fähigkeit aus, sich im Laufe der Zeit im Gewebe zu zersetzen. Sie kommen in der Orthopädie, Wundversorgung oder bei Drug Delivery zum Einsatz. Die gebräuchlichsten Materialien sind Polylactic acid (PLA), Polyglycolic acid (PGA) und ihre Co-Polymere sowie Polycaprolactone (PCL). Die Schutzschicht kann darüber hinaus auch Gelatine beinhalten oder im Wesentlichen aus Gelatine bestehen.

Der Einsatz dieser Polyester in der Schutzschicht ist besonders bevorzugt, da sie besonders leicht durch einfache Hydrolyse degradieren, die Hydrolyseprodukte durch normale metabolische Prozesse resorbiert werden, und sie es außerdem ermöglichen, die Abbaurate gezielt einzustellen. Bestimmende Faktoren für die Abbaurate sind neben der genauen molekularen Struktur das Verhältnis der Co-Polymere zueinander, das Molekulargewicht und ggf. auch das Herstellungsverfahren selbst. Die Abbauraten dieser Polymere liegen im Bereich von 1 bis 24 Monaten.

Einen Überblick über im Rahmen der vorliegenden Erfindung verwendbare Materialien liefern P. A. Gunatillake und R. Adhikari: "BIODEGRADABLE SYNTHETIC POLYMERS FOR TISSUE ENGINEERING", in European Cells and Materials (2003), 5, Seiten 1-16. Je nach Einsatzart des neuen Retina-Implantats sowie der gewünschten Eindringgeschwindigkeit der Elektroden in das entsprechende Gewebe können die bekannten Materialien so zusammengestellt werden, dass sie die entsprechende Abbaurate aufweisen. Vor diesem Hintergrund wird die Offenbarung der Veröffentlichung von Gunatillake und Adhikari a.a.O. ausdrücklich zum Gegenstand der vorliegenden Erfindung gemacht:

Bioresorbierbare Materialien für biomedizinische Anwendungen sowie biodegradierbare Polyester sind darüber hinaus im Stand der Technik vielfach beschrieben. Im Gegensatz zu reinen Polymeren weisen beispielsweise Implantate aus biodegradierbaren Polyestern (Poly(L-lactid) und Poly(D,L-lactid)) sowie amorphem, carbonathaltigem Calciumphosphat bzw. Calciumcarbonat den Vorteil auf, dass sie beim Abbau keine sauren Produkte freisetzen sondern einen physiologischen pH-Wert aufweisen, da die entstehenden Säuren durch den anorganischen Füllstoff abgepuffert werden; siehe beispielsweise C. Schiller: "NEUE MATERIALIEN IM KOPF: SCHÄDELIMPLANTAT LÖST SICH VON INNEN HER AUF", in www.uni-protokolle.de /nachrichten/id/25555/.

Gut geeignet sind ferner bioresorbierbare Schichten aus Metall bzw. Metall-Legierungen, wie z.B. Magnesium und Magnesium-Legierung (siehe www.unics.unihannover.de/analytik/Forschung/Bioresorbierbare%20Iplantate.pdf).

Aus der DE 100 28 522 A1 ist in diesem Zusammenhang eine biodegradable Neuroelektrode bekannt, die mit einem mechanischen Stützelement aus biodegradablem Material versehen ist, um das mechanisch selbst nicht stabile Implantat während der Implantation handhaben zu können.

In vergleichbarer Weise offenbart die US 6,792,315 B2 eine in das Augenlid implantierbare Elektrodenanordnung, die auf einem Träger aus biodegradierbarem Material angeordnet ist, der sich nach der Implantation auflöst. Während der Implantation wird auch hier die Elektrodenanordnung durch den Träger stabilisiert, so dass sie besser gehandhabt werden kann.

Weder in der DE 100 28 522 A1 noch in der US 6,792,315 B2 bedeckt die biodegradierbare Struktur die Elektroden, die erfüllt jeweils nur eine mechanische Stützfunktion. Während der Implantation sind die Elektroden bei diesen bekannten Vorrichtungen nicht vor Beschädigungen geschützt, sie gelangen unmittelbar während des Eingriffs in Kontakt mit dem Gewebe.

Die Verwendung von biodegradablen und/oder bioresorbierbaren Materialien als sozusagen vorübergehende Schutzschicht auf einem Array von Nadelelektroden bei einer implantierbaren Vorrichtung ist im Stand der Technik jedoch bisher nicht beschrieben.

In einer Weiterbildung ist es bevorzugt, wenn in die Schutzschicht biologisch aktive Substanzen eingelagert sind, die bei der Auflösung der Schutzschicht freigesetzt werden.

Bei dieser Maßnahme ist von Vorteil, dass bei dem Abbau der Schutzschicht gleichzeitig aktive Substanzen in das umgebende Gewebe abgegeben werden können, die beispielsweise die Narbenbildung verhindern oder entzündungshemmend wirken.

Besonders bevorzugt ist es, wenn die aktive Substanz ein entzündungshemmendes Steroid ist, wie es beispielsweise aus der eingangs erwähnten EP 0 388 480 A1 bekannt ist. Vorzugsweise wird als Steroid Dexamethason und/oder Cortison eingesetzt.

Allgemein ist es bevorzugt, wenn die Elektrode eine Grundelektrode umfasst, von der eine Vielzahl von Nadelelektroden abstehen.

Bei dieser Maßnahme ist von Vorteil, dass eine mechanisch engere Kopplung mit den zu stimulierenden Zielzellen erreicht wird als bei der Grundelektrode selbst. Ferner ist eine niedrigere Reizschwelle erforderlich, denn die Oberfläche der Grundelektrode erstreckt sich sozusagen zu den Zielzellen wie die im biologischen Gewebe an Nervenzellen vorhandenen Dendriten, wobei die Elektroden auch eine ähnliche Größenordnung aufweisen.

Es handelt sich damit sozusagen um eine dreidimensionale Nanoelektrode, bei der die Vielzahl von Nadelelektroden, die sozusagen einen nanoskaligen Teil der Elektrode darstellen, möglichst schonend in die angrenzende Zellschicht eindringen kann.

Derartige Nanoelektrodenstrukturen aus einer planaren Grundelektrode mit einer Vielzahl von abstehenden Nadelelektroden können beispielsweise auf an sich in anderem Zusammenhang bekannte Art und Weise mit einem Elektronenstrahlschreiber durch Elektronenstrahlbelichtung entsprechender Masken und anschließendes Plasma-Ätzen hergestellt oder mit einem FIB- (Focussed Ion Beam) Gerät direkt geätzt oder reaktiv abgeschieden werden.

Andererseits ist es auch möglich, die dreidimensionale Nanoelektrodenstruktur in UV-härtbarem Polymer auszubilden, das im Spincoating-Verfahren auf eine Substratoberfläche aufgebracht wird. In das Polymer wird dazu ein Nanostempel angedrückt, danach das Polymer ausgehärtet und schließlich entformt. Die so in das Polymer im Nanoprint-Verfahren eingebrachte Nanostruktur wird anschließend mit dem Elektrodenmaterial durch reaktives Sputtern mit TiN, Ir oder IrO beschichtet. Nach dem Aufbringen des Steroids kann die Struktur dann mit dem biodegradablen Material beschichtet werden.

Der Nanostempel muss also nur einmal beispielsweise durch Elektronenstrahlschreiben hergestellt werden, wobei mit dem Nanostempel dann im Prinzip beliebig viele Nanostrukturen hergestellt werden können.

Allgemein ist es noch bevorzugt, wenn die Elektrode aus einem flexiblen Material gefertigt ist.

Bei dieser Maßnahme ist von Vorteil, dass aufgrund der anfänglich auf dem Grundkörper vorgesehenen, die Elektroden abdeckenden Schutzschicht auch flexible Elektroden für Implantate verwendet werden können, ohne dass die Gefahr besteht, dass bei der Handhabung der Vorrichtung die flexiblen Elektroden beschädigt werden. Flexible Elektroden haben darüber hinaus den Vorteil, dass sie bei der Auflösung der Schutzschicht selbst bestimmten Strukturen im Gewebe ausweichen können, so dass sie eine schonende Penetration ermöglichen.

Die Flexibilität des Materials kann dabei sowohl durch Materialeigenschaften als auch durch die Abmaße des Materials erreicht werden, besonders dünne Nadelelektroden weisen beispielsweise auch dann eine gewisse Flexibilität auf, wenn sie aus Metall gefertigt sind.

Allgemein ist es dann noch bevorzugt, wenn auf dem Grundkörper zumindest eine planare Elektrode vorgesehen ist.

Diese planare Elektrode kann in an sich bekannter Weise der Masseverbindung des Retina-Implantats mit dem umgebenden Gewebe dienen. Dabei ist es einerseits möglich, die nadelförmigen Elektroden und die planare Elektrode auf unterschiedlichen Seiten des Grundkörpers anzuordnen, wobei es auch möglich ist, beide Elektrodenarten auf der selben Seite des Grundkörpers anzuordnen.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: in schematischer Darstellung einer implantierbaren Vorrichtung, hier ein Retina-Implantat, in nicht maßstabgetreuer Darstellung;
- Fig. 2: eine schematische Darstellung eines menschlichen Auges, in das das Retina-Implantat gemäß Fig. 1 eingesetzt ist, ebenfalls nicht maßstabgetreu;
- Fig. 3: eine schematische Darstellung des Retina-Implantates aus Fig. 1;
- Fig. 4: eine schematische Darstellung der Implantation des Retina-Implantates aus Fig. 3 in umgebendes Gewebe; und
- Fig. 5: eine schematische Darstellung der Kontaktierung eines Elektrodenarrays mit einem Gewebe *ex vivo.*

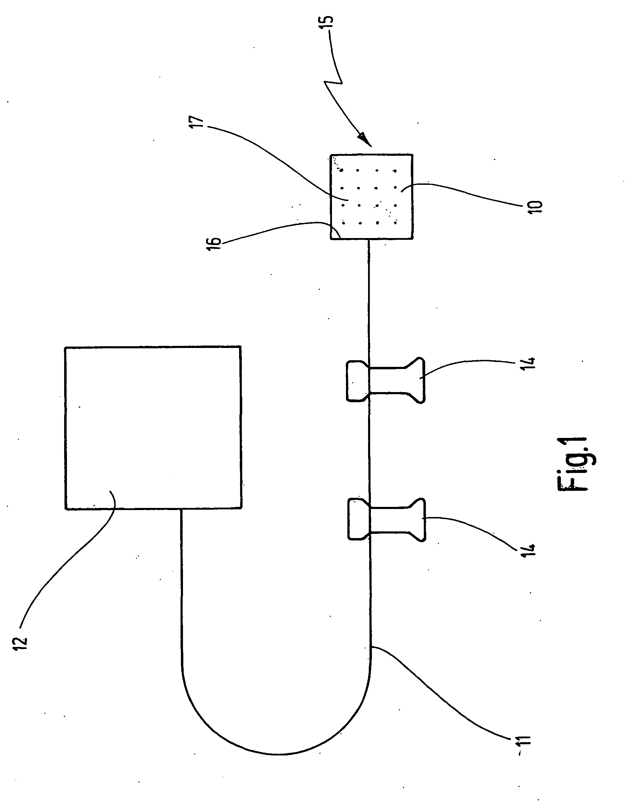

In Fig. 1 ist schematisch eine implantierbare Vorrichtung 10 dargestellt, wobei die Abmaße nicht maßstabgetreu wiedergegeben sind. Die Vorrichtung 10 ist über ein Kabel 11 mit einer Versorgungseinheit 12 verbunden, die die Vorrichtung 10 mit elektrischer Energie und mit Steuersignalen versorgt. An dem Kabel 11 sind Befestigungslaschen 14 vorgesehen, mit denen das Kabel am Körper der Person befestigt werden kann, der das Implantat 10 eingepflanzt wird.

Die Vorrichtung 10 ist ein aktives Retina-Implantat 15, das als Grundkörper eine Folie 16 aufweist, auf der Elektroden 17 zur Abgabe von Stimulationssignalen an erregbare Zellen angeordnet sind.

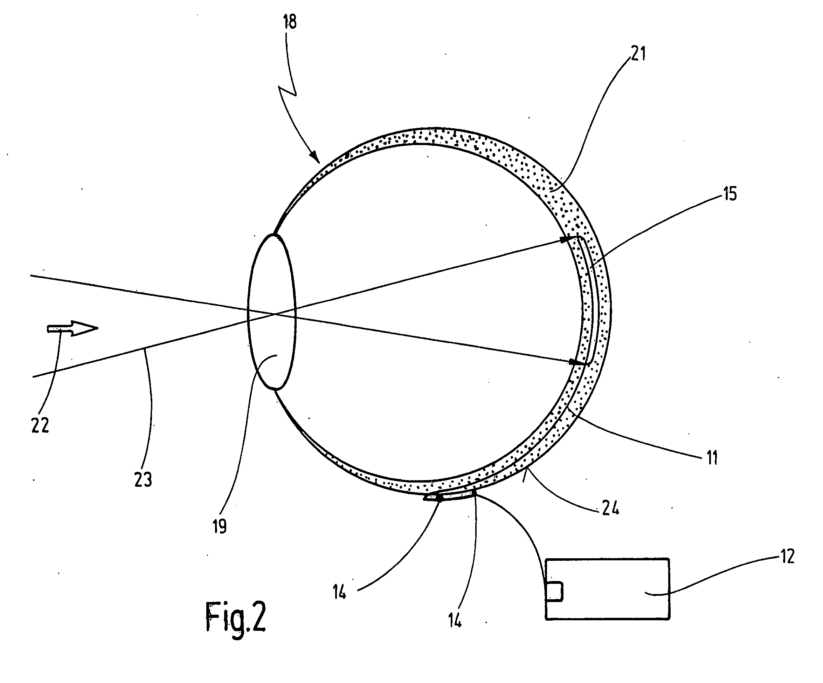

Das Retina-Implantat 15 aus Fig. 1 ist dazu bestimmt, in ein menschliches Auge 18 implantiert zu werden, das in Fig. 2 sehr schematisch dargestellt ist. Der Einfachheit halber sind nur die Linse 19 sowie die Retina 21 gezeigt, in die das Implantat 15 eingepflanzt wurde. Das Implantat 15 wird dabei vorzugsweise in den so genannten subretinalen Raum eingebracht, der sich zwischen dem Pigment-Epithel und der Fotorezeptorschicht bildet. Sofern die Fotorezeptorschicht degeneriert oder verloren ist, bildet sich der subretinale Raum zwischen dem Pigment-Epithel und der Schicht der Bipolar- und Horizontalzellen. Das Retina-Implantat 15 wird dabei so platziert, dass über die in Fig. 1 gezeigten Elektroden 17 Stimulationssignale auf Zellen in der Retina 21 ausgeübt werden können.

Durch einen Pfeil 22 angedeutetes sichtbares Licht, dessen Strahlengang bei 23 zu sehen ist, wird über die Linse 19 auf das Implantat 15 geleitet, wo das sichtbare Licht 2 in elektrische Signale umgewandelt wird, die in Stimulationssignale gewandelt werden.

Es ist zu erkennen, dass das Kabel 11 seitlich aus dem Auge herausgeführt und dort außen auf der Sklera 24 mit den Befestigungslaschen 14 befestigt wird, bevor das Kabel weiter zu der externen Versorgungseinheit 12 führt.

Die Versorgungseinheit 12 wird dann in nicht näher gezeigter Art und Weise außerhalb des Auges beispielsweise am Schädel des Patienten befestigt. Über die Versorgungseinheit 12 wird elektrische Energie zu dem Implantat 10 gesandt, wobei gleichzeitig auch Steuersignale übermittelt werden können, die die Funktionsweise des Implantates so beeinflussen, wie dies beispielsweise in der eingangs erwähnten WO 2005/000395 A1 beschrieben ist, deren Inhalt hiermit zum Gegenstand der vorliegenden Anmeldung gemacht wird.

Es sei noch erwähnt, dass die Abmaße insbesondere des Retina-Implantates 15, der Befestigungslaschen 14 sowie der externen Versorgungseinheit 12 in den Fig. 1 und 2 weder maßstäblich noch in richtiger Größenrelation zueinander dargestellt sind.

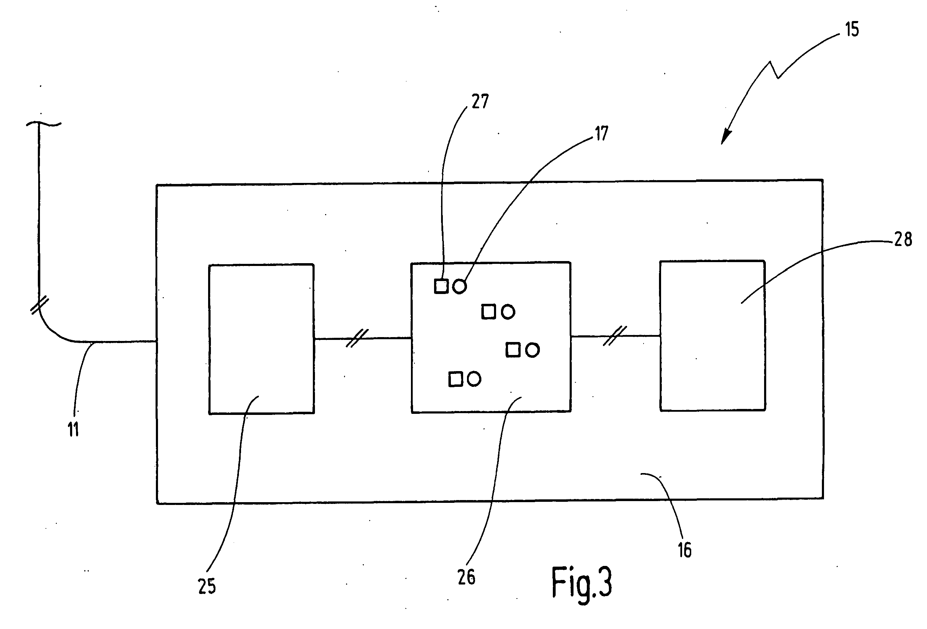

In Fig. 3 ist schematisch der Aufbau des aktiven Retina-Implantates 15 aus Fig. 1 gezeigt. Auf der Folie 16 ist zunächst eine Eingangsstufe 25 zu erkennen, der über das Kabel 11 von außen externe Fremdenergie zugeführt wird. Die Eingangsstufe 15 ist mit einer Sensoreinheit 26 verbunden, die in diesem Falle eine Vielzahl von Bildzellen 27 aufweist, die einfallendes sichtbares Licht in elektrische Signale umwandelt, die dann über die neben den jeweiligen Bildzellen angedeuteten Elektroden 17 an Nervenzellen der Retina abgegeben werden.

Die Verarbeitung der von den Bildzellen 27 erzeugten Nutzsignale erfolgt in einer Ausgangsstufe 28, die die entsprechenden Stimulationssignale erzeugt, die dann zurück zu der Sensoreinheit 26 bzw. den Elektroden 17 geführt werden.

In diesem Zusammenhang sei darauf hingewiesen, dass die Fig. 3 lediglich eine schematische, den logischen Aufbau wiedergebende Darstellung des Retina-Implantates 15 ist, die tatsächliche geometrische Anordnung der einzelnen Komponenten kann dazu führen, dass beispielsweise jede Bildzelle 27 in unmittelbarer Nachbarschaft eine Ausgangsstufe aufweist.

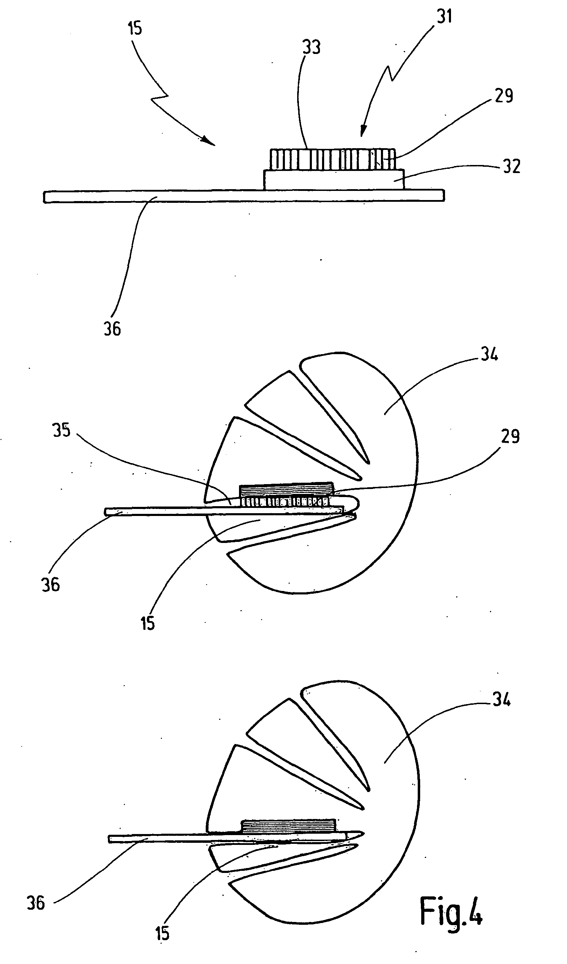

Die Elektroden 17 können beispielsweise als Nadelelektroden 29 ausgebildet und in einem gesonderten Array 31 auf einem Grundkörper 32 angeordnet sein, wie dies jetzt in Fig. 4 oben gezeigt ist. Die Nadelelektroden 29 haben bspw. einen Durchmesser von 10 µm und eine Höhe über dem Grundköper 32 von 70 µm, wobei sie sich nach oben hin verjüngen.

Die Elektroden 17 sind dabei von einer Schutzschicht 33 abgedeckt, die aus einem biodegradablen und/oder bioresorbierbaren Material mit einer definierten Abbaurate besteht. In die Schutzschicht 33 sind biologisch aktive Substanzen eingelagert, die bei der Auflösung der Schutzschicht 33 freigesetzt werden.

Die biologisch aktiven Substanzen wirken entzündungshemmend, wobei sie auch fördernd oder hemmend auf das Zellwachstum wirken. In vielen Fällen wird ein Steroid wie Cortison und/oder Dexamethason in die Schutzschicht 33 eingebettet.

Dieser Grundkörper 32 wird jetzt in bei 34 angedeutetes Gewebe implantiert, wozu er in einen Einschnitt 35 eingeführt wird.

Der Grundkörper 32 ist dabei auf einem Träger 36 gehalten, über den das Retina-Implantat 15 jetzt in den Einschnitt 35 hineingeschoben wird, wie dies in der mittleren Abbildung der Fig. 4 gezeigt ist.

Der Einschnitt 35 drückt jetzt auf das Implantat 15, wodurch die Schutzschicht 33 in Kontakt mit dem Gewebe 34 gelangt und sich allmählich abbaut. In dem Maße, wie sich die Schutzschicht 33 abbaut, dringen die Nadelelektroden 29 in das Gewebe 34 ein, bis die Nadelelektroden 29 schließlich vollständig in dem Gewebe 34 aufgenommen sind, wie dies in Fig. 4 unten gezeigt ist.

Während des Einschiebens des Implantates 15 in das Gewebe 34 sind die Elektroden 17 also durch die Schutzschicht 33 geschützt, wobei gleichzeitig auch Strukturen des Gewebes 34 beim Einschieben nicht verletzt werden können.

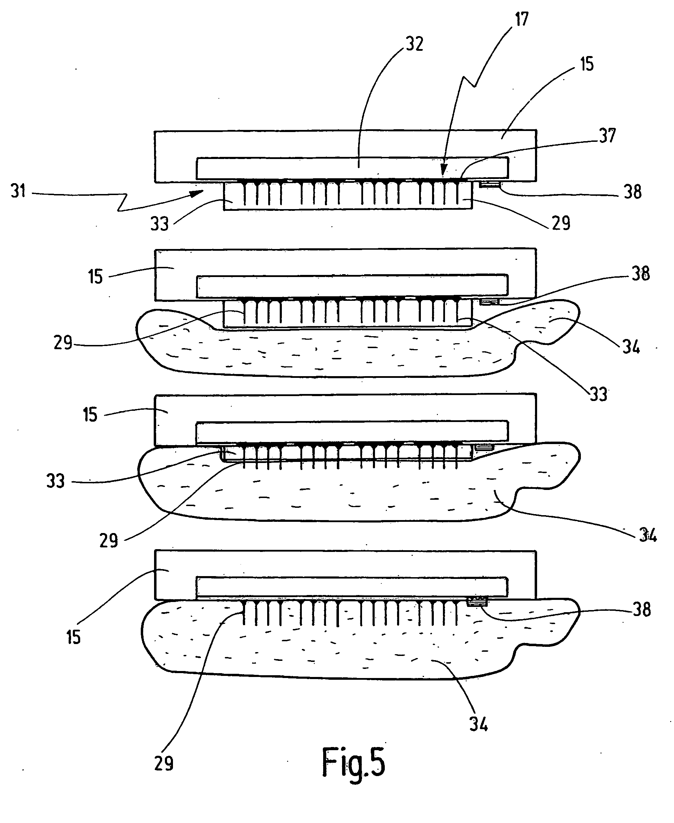

In Fig. 5 ist noch einmal schematisch das Eindringen der Nadelelektroden 29 in ein Gewebe 34 gezeigt, das *ex vivo* vorliegt. Auch hier kann das Retina Implantat 15 verwendet werden, wenn es bspw. *ex vivo* getestet wird.

In Fig. 5 oben ist das Implantat 15 in einer schematischen Seitenansicht gezeigt, wobei zu erkennen ist, dass in dem Array 31 von Elektroden 17 jede Elektrode 17 jeweils eine Grundelektrode 37 umfasst, von der mehrere Nadelelektroden 29 abstehen. Auf diese Weise ist jede Elektrode 17, die das Signal einer Bildzelle an die Retina liefert, mit mehreren Nadelelektroden 29 versehen, so dass eine gute mechanische und elektrische Anbindung der Elektrode 17 an das Gewebe 34 erfolgt.

Ferner ist schematisch auf dem Implantat in Fig. 5 noch eine planare Elektrode 38 zu sehen, die der Masseanbindung des Implantates 15 zu dem Gewebe 34 dient, wie dies an sich bekannt ist.

Die Herstellung des Elektrodenarrays 31 aus Fig. 5 mit den Grundelektroden 37 und davon abstehenden Nadelelektroden 29 erfolgt so, wie dies in der Beschreibungseinleitung bereits ausführlich beschrieben wurde, nämlich entweder über Elektronenstrahlschreiben mit nachfolgendem Plasma-Ätzen, oder im Nanoimprint-Verfahren, bei dem eine Nanostruktur in ein UV-härtbares Polymer eingebracht wird, wobei auf die so erzeugte Nanostruktur anschließend das Elektrodenmaterial aus TiN, Ir oder IrO durch reaktives Sputtern aufgebracht wird. Anschließend wird das Steroid aufgebracht, bevor dann die biodegradable Schutzschicht 33 aufgebracht wird.

Das Retina Implantat wird dabei bspw. durch sein Gewicht oder eine von außen, also in Fig. 5 von oben auf das Gewebe gedrückt, so dass sich die Nadelelektroden 29 langsam in das Gewebe 34 vorschieben, wenn die Schutzschicht 33 abgebaut wird.

Der Abbau der Schutzschicht 33 kann allein durch den Kontakt mit dem Gewebe 34 bewirkt werden, wobei es auch möglich ist, den Abbau durch chemische, biologische oder physikalische Prozesse auszulösen.

## Patentansprüche

1. Aktives Retina-Implantat (15) mit einer Vielzahl von Bildzellen (27), die einfallendes Licht (22) in elektrische Signale umwandeln, die über Elektroden (17) an umgebendes Gewebe (21, 34) abgegeben werden, wobei die Elektroden (17) Nadelelektroden (29) sind, die als Array (31) auf einem Grundkörper (15, 32) angeordnet sind, **dadurch gekennzeichnet, dass** die Elektroden (17) von einer Schutzschicht (33) abgedeckt sind und die Schutzschicht (33) derart beschaffen ist, dass sie sich nach Kontakt mit dem Gewebe (21, 34) auf definierte Weise zumindest so weit auflöst, dass die Elektroden (17) in unmittelbaren Kontakt mit dem Gewebe (21, 34) gelangen, wobei die Schutzschicht (33) biodegradable und/oder bioresorbierbare Materialien mit einer definierten Abbaurate umfasst.

2. Retina-Implantat nach Anspruch 1" **dadurch gekennzeichnet, dass** die Schutzschicht (33) Polyglycolic acid, L-Polylactic acid, D,L-Polylactic acid, Polycaprolactone, deren Co-Polymere, Gelatine, und/oder biologisch abbaubare Metalle bzw. Metall-Legierungen, wie z.B. Magnesium und Magnesium-Legierungen umfasst.

3. Retina-Implantat nach Anspruch 1 oder 2, **dadurch gekennzeichnet, dass** in die Schutzschicht (33) biologisch aktive Substanzen eingelagert sind, die bei der Auflösung der Schutzschicht (33) freigesetzt werden.

4. Retina-Implantat nach Anspruch 3, **dadurch gekennzeichnet, dass** die biologisch aktive Substanz entzündungshemmend ist und/oder fördernd oder hemmend auf das Zellwachstum wirkt, vorzugsweise ein Steroid umfasst, das weiter vorzugsweise Cortison und/oder Dexamethason umfasst.

5. Retina-Implantat nach einem der Ansprüche 1 bis 4, **dadurch gekennzeichnet, dass** die Elektroden (17) eine Grundelektrode (37) umfassen, von der eine Vielzahl von Nadelelektroden (29) abstehen.

6. Retina-Implantat nach einem der Ansprüche 1 bis 5, **dadurch gekennzeichnet, dass** die Elektroden (17) aus einem flexiblen Material gefertigt sind.

7. Retina-Implantat nach einem der Ansprüche 1 bis 6, **dadurch gekennzeichnet, dass** auf dem Grundkörper (32) zumindest eine planare Elektrode (38) vorgesehen ist.

## Claims

1. An active retinal implant (15) comprising a multiplicity of image cells (27) that convert incident light (22) into electrical signals, which electrical signals are delivered via electrodes (17) to surrounding tissue (21, 34), wherein said electrodes (17) are needle electrodes (29) arranged as an array (31) on a base body (15, 32), **characterized in that** said electrodes (17) are covered by a protective layer (33), said protective layer (33) being such that, after contact with the tissue (21, 34), it decomposes in a defined manner and at least to such an extent that said electrodes (17) come into direct contact with said tissue (21, 34), and wherein the protective layer (33) comprises biodegradable and/or bioabsorbable materials having a defined rate of degradation.

2. The retinal implant of Claim 1, **characterized in that** the protective layer (33) comprises polyglycolic acid, L-polylactic acid, D,L-polylactic acid, polycaprolactone, copolymers thereof, gelatin, and/or biodegradable metals and metal alloys, like e.g. magnesium and magnesium alloys.

3. The retinal implant of Claim 1 or 2, **characterized in that** the protective layer (33) incorporates biologically active substances that are released when the protective layer (33) decomposes.

4. The retinal implant of Claim 3, **characterized in that** the biologically active substance is anti-inflammatory and/or acts promoting or inhibiting on cell growth, preferably comprises a steroid, which further preferably comprises cortisone and/or dexamethasone.

5. The retinal implant of anyone of Claims 1 to 4, **characterized in** at the electrode (17) comprises a base electrode (37), a multiplicity of needle electrodes (29) projecting from said base electrode.

6. The retinal implant of anyone of Claims 1 to 5, **characterized in that** the electrode (17) is made from a flexible material.

7. The retinal implant of anyone of Claims 1 to 6, **characterized in that** at least one planar electrode (38) is provided on the base body (32).

## Revendications

1. Implant rétinien actif (15) comportant une multiplicité de cellules d'image (27) qui convertissent une lumière incidente (22) en signaux électriques qui sont distribués par des électrodes (17) au tissu environnant (21, 34), les électrodes (17) étant des électrodes à aiguille (29) qui sont disposées sous forme de barrette (31) sur un corps de base (15, 32), **caractérisé en ce que** les électrodes (17) sont recouvertes d'une couche protectrice (33) et la couche protectrice (33) est conçue de telle sorte qu'elle se dissout après un contact avec le tissu (21, 34) de façon définie au moins aussi longtemps que les électrodes (17) sont en contact direct avec le tissu (21, 34), la couche protectrice (33) comprenant des matériaux biodégradables et/ou biorésorbables présentant une vitesse de décomposition définie.

2. Implant rétinien selon la revendication 1, **caractérisé en ce que** la couche protectrice (33) comprend de l'acide polyglycolique, de l'acide L-polylactique, de l'acide D,L-polylactique, de la polycaprolactone, leurs copolymères, de la gélatine et/ou des métaux ou alliages de métaux biodégradables tels que par exemple le magnésium et les alliages du magnésium.

3. Implant rétinien selon la revendication 1 ou 2, **caractérisé en ce que** dans la couche protectrice (33) sont stockées des substances biologiquement actives qui sont libérées lors de la dissolution de la couche protectrice (33).

4. Implant rétinien selon la revendication 3, **caractérisé en ce que** la substance biologiquement active est anti-inflammatoire et/ou agit de façon à stimuler ou inhiber la croissance cellulaire, de préférence elle comprend un stéroïde qui comprend en outre de préférence de la cortisone et/ou de la dexaméthasone.

5. Implant rétinien selon l'une des revendications 1 à 4, **caractérisé en ce que** les électrodes (17) comprennent une électrode de base (37) de laquelle part une multiplicité d'électrodes en aiguille (29).

6. Implant rétinien selon l'une des revendications 1 à 5, **caractérisé en ce que** les électrodes (17) sont en un matériau flexible.

7. Implant rétinien selon l'une des revendications 1 à 6, **caractérisé en ce que**, sur le corps de base (32) est prévue au moins une électrode planaire (38).
